# FASCICULE DE BREVET EUROPEEN

(11) **EP 2 343 039 B1**
(45) Date de publication et mention de la délivrance du brevet: **26.10.2016**
(21) Numéro de dépôt: 10195453.5
(22) Date de dépôt: 16.12.2010
(51) Int. Cl.: A61K 8/39, A61K 8/44, A61K 8/46, A61K 8/58, A61Q 5/02, A61Q 5/12

(54) **Composition cosmétique comprenant au moins un compose organique du silicium, au moins deux tensioactifs anioniques et au moins un tensioactif amphotère**
Kosmetische Zusammensetzung, die mindestens eine organische Komponente aus Silizium, mindestens zwei anionische Tenside und mindestens ein amphoteres Tensid enthält
Cosmetic composition containing at least one organic silicon compound, at least two anionic surface-active agents and at least one amphoteric surface-active agent

(30) Priorité: 23.12.2009 FR 0959486
(43) Date de publication de la demande: 13.07.2011
(73) Titulaire: L'Oréal, 75008 Paris (FR)
(72) Inventeur: Aires, Carine, 75014, Paris (FR); Drillon, Damien, 75008, Paris (FR); Viravau, Valérie, 75001, Paris (FR)
(74) Mandataire: Dossmann, Gérard

(56) Documents cités:
- EP-A1- 1 726 293
- FR-A1- 2 789 896

## Description

La présente invention concerne une composition cosmétique destinée au nettoyage et au conditionnement des fibres kératiniques, en particulier des fibres kératiniques humaines telles que les cheveux, comprenant, dans un milieu cosmétiquement acceptable, un ou plusieurs composés organiques du silicium, un ou plusieurs tensioactifs anioniques sulfates ou sulfonates, un ou plusieurs tensioactifs anioniques carboxyliques particuliers et un ou plusieurs tensioactifs amphotères. La présente invention concerne également un procédé de traitement cosmétique des fibres kératiniques ainsi qu'une utilisation mettant en oeuvre ladite composition cosmétique.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base présentent certes un bon pouvoir lavant mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (c'est-à-dire les cheveux qui se trouvent généralement abîmés ou fragilisés par l'action des agents atmosphériques extérieurs tels que la lumière et les intempéries, et/ou des traitements mécaniques ou chimiques tels que le brossage, le peignage, les teintures, les décolorations, les permanentes et/ou les défrisages), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents de conditionnement peuvent bien entendu également améliorer le comportement cosmétique des cheveux naturels.

Dans ce but, on a déjà proposé d'utiliser des composés organiques cosmétiquement actifs tels que des polymères cationiques et des silicones en tant qu'agents de conditionnement, dans des compositions cosmétiques détergentes tels que des shampooings, pour conférer aux cheveux des propriétés cosmétiques satisfaisantes, en particulier de la brillance, de la douceur, de la souplesse, de la légèreté, un toucher naturel ainsi qu'une aptitude au démêlage améliorée.

Cependant, l'emploi de ces composés dans des compositions cosmétiques de lavage et de conditionnement ne procure pas aux cheveux des propriétés coiffantes satisfaisantes et durables. En effet, ces compositions procurent généralement des effets coiffants, tels que des effets de maintien, de corps et/ou de discipline des cheveux, qui restent insuffisants et qui ont tendance à s'estomper après un lavage des cheveux avec un shampooing classique.

Or on a constaté que les consommateurs sont de plus en plus à la recherche de compositions de lavage qui soient non seulement capables de conditionner les cheveux de manière convenable mais également capables de procurer des effets coiffants satisfaisants et durables.

Ainsi des compositions destinées au lavage et au conditionnement des cheveux qui comprennent des composés organiques du silicium, tel que le 3-aminopropyltriéthoxysilane, ont été développées afin de pouvoir répondre à ces exigences. Ces compositions de lavage permettent de conditionner les cheveux, notamment en leur apportant un toucher doux satisfaisant, tout en conférant des effets coiffants marqués et durables.

De plus, ces compositions se sont révélées particulièrement avantageuses car elles permettent de faciliter la mise en forme des cheveux fins et de conférer des effets coiffants intéressants aux cheveux bouclés ou frisés, notamment en améliorant le dessin et le contrôle des boucles.

Toutefois, les compositions de lavage comprenant de tels composés organiques du silicium présentent généralement l'inconvénient d'évoluer sensiblement au cours du temps dans des conditions normales de stockage en fonction de la température, notamment au niveau de leur viscosité et de leur aspect visuel. En d'autres termes, ces compositions ne s'avèrent pas stables ce qui se traduit le plus souvent par un aspect visuel trouble ainsi que par une texture non satisfaisante au stockage.

En effet, il a été constaté que les composés organiques du silicium, tel que le 3-aminopropyltriéthoxysilane, n'étaient pas compatibles chimiquement avec la totalité des tensioactifs, notamment des tensioactifs anioniques, qui peuvent être présents dans les compositions de lavage, ce qui engendre les problèmes de stabilité rencontrés.

Par ailleurs, on a observé que l'introduction de certains composés organiques du silicium, en particulier les dérivés aminés tels que le 3-aminopropyltriéthoxysilane, dans des compositions de lavage, qui présentent généralement un pH allant de 4 à 7, engendre également des problèmes de stabilité du fait du caractère alcalin de ces composés.

Il existe donc un réel besoin de mettre au point des compositions cosmétiques destinées au nettoyage et au conditionnement des fibres kératiniques contenant des composés organiques du silicium, ces compositions ne présentant pas l'ensemble des inconvénients décrits ci-dessus, c'est-à-dire qui sont stables dans le temps et qui permettent de conditionner les cheveux de manière satisfaisante tout en apportant des effets coiffants puissants durables, notamment en termes de masse, de corps, de texturisation des cheveux.

La demanderesse a découvert, de façon surprenante, qu'il était possible de formuler des compositions détergentes et conditionnantes des fibres kératiniques, ayant les propriétés recherchées, comprenant un ou plusieurs composés organiques du silicium tels que définis ci-après, un ou plusieurs tensioactifs anioniques sulfates ou sulfonates, un ou plusieurs tensioactifs anioniques carboxyliques différents des précédents choisis parmi les acides alkyl éther carboxyliques et leurs sels et un ou plusieurs tensioactifs amphotères.

En effet, il a été constaté que l'utilisation d'un ou plusieurs tensioactifs amphotères et d'un ou plusieurs tensioactifs anioniques carboxyliques choisis parmi les acides alkyl éther carboxyliques et leurs sels, dans des compositions cosmétiques comprenant un ou plusieurs composés organiques du silicium et un ou plusieurs tensioactifs anioniques sulfates ou sulfonates, permet de les rendre stables au stockage aussi bien à température ambiante (20-25°C) qu'à 45°C, notamment au niveau de leur aspect visuel et de leur viscosité.

Par « stables » au sens de la présente invention, on entend que l'aspect visuel ainsi que la viscosité de ces compositions n'évoluent pas sensiblement au cours du temps dans des conditions de tests de stockage, par exemple pendant les 2 mois à température ambiante (20°C-25°C), et/ou à 45°C et/ou à 4°C, qui suivent leur fabrication.

De plus, les compositions conformes à l'invention conduisent à un traitement satisfaisant des cheveux leur conférant ainsi un toucher doux satisfaisant, une aptitude au démêlage améliorée, de la douceur et la souplesse.

Par ailleurs, les compositions conformes à la présente invention apportent des effets coiffants puissants, notamment en leur apportant de la masse, du corps et/ou de la discipline et ceci de manière durable.

De plus, les compositions selon l'invention permettent de faciliter la mise en forme des cheveux, en particulier des cheveux fins, et de conférer des effets coiffants améliorés aux cheveux bouclés, notamment en terme de dessin et de contrôle des boucles, et ceci de manière durable.

La présente invention concerne notamment une composition cosmétique détergente et conditionnante des fibres kératiniques, en particulier des fibres kératiniques humaines telles que les cheveux, comprenant dans un milieu cosmétiquement acceptable :
(i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques sulfates ou sulfonates,
(iii) un ou plusieurs tensioactifs anioniques carboxyliques différents des tensioactifs (ii) choisis parmi les acides alkyléther carboxyliques et leurs sels, et
(iv) un ou plusieurs tensioactifs amphotères.

La présente invention concerne également un procédé de traitement cosmétique des cheveux, en particulier de lavage et de conditionnement, comprenant l'application sur lesdites fibres de la composition selon l'invention.

Elle concerne aussi l'utilisation de la composition selon l'invention comme shampoing pour le lavage et le conditionnement des cheveux.

D'autres objets et caractéristiques, aspects et avantages de l'invention apparaîtront encore plus clairement à la lecture de la description et des exemples qui suivent.

Les composés organiques du silicium (i) utilisés dans la composition selon l'invention sont choisis parmi les organosilanes comprenant un atome de silicium et les organosiloxanes comportant deux ou trois atomes de silicium, de préférence deux atomes de silicium. Ils doivent en outre comporter une ou plusieurs fonctions chimiques basiques, et de préférence une seule fonction chimique basique. La fonction chimique basique peut correspondre à toute fonction conférant un caractère basique au composé de silicium et est de préférence une fonction amine telle qu'une fonction amine primaire, secondaire ou tertiaire. Les composés du silicium selon l'invention, peuvent comporter éventuellement d'autres fonctions, telles que, par exemple, une fonction acide ou une fonction halogène.

Le ou les composés organiques du silicium (i) utilisés dans la composition selon invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les organosilanes utilisées dans la composition selon l'invention sont choisis parmi les composés de formule (I) : 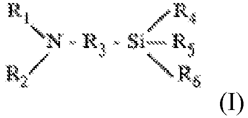 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R', R" et R"' étant différents de l'hydrogène.

De préférence, les groupes R₁, R₂, R', R'₁, R'₂, R'₃, R" et R'" sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

Selon un autre mode de réalisation particulier, le ou les organosiloxanes utilisées dans la composition selon l'invention sont choisis parmi les composés de formule (II) : 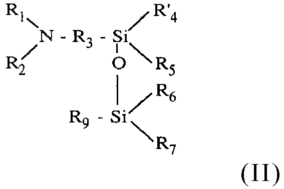 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ; R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR''', OR₁₀ ou OR₈.

De préférence, les groupes R''₁, R''₂, R₈ ou R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

En particulier, l'atome d'halogène est un atome de chlore.

Le ou les composés organiques du silicium utilisés dans la composition selon invention sont de préférence des organosilanes choisis parmi les composés de formule (III) : 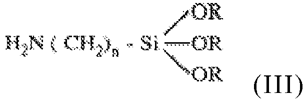 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium (i) présent dans la composition selon l'invention est le 3-aminopropyltriéthoxysilane.

Le ou les composés organiques du silicium (i) peuvent être présents dans la composition selon l'invention dans une teneur allant de 0,01 à 10% en poids, de préférence dans une teneur en poids allant de 0,1 à 5% en poids, et plus préférentiellement dans une teneur en poids allant de 0,2 à 2% en poids, par rapport au poids total de la composition.

Comme indiqué précédemment, la composition cosmétique selon la présente invention contient en outre un ou plusieurs tensioactifs sulfates ou sulfonates (ii).

Le ou les tensioactifs anioniques sulfates ou sulfonates (ii) utilisés dans les compositions selon l'invention sont des tensioactifs anioniques comportant un ou plusieurs fonctions sulfates (-OSO₃H ou -OSO₃⁻) et/ou une ou plusieurs fonctions sulfonates (-SO₃H ou -SO₃⁻).

Le ou les tensioactifs anioniques sulfates ou sulfonates (ii) utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10, mieux de 2 à 5.

On utilise de préférence un agent tensioactif anionique choisi parmi les alkyl(C₁₂-C₁₄) sulfates de sodium, de triéthanolamine, de magnésium ou d'ammonium, les alkyl (C₁₂-C₁₄)éthersulfates de sodium, d'ammonium ou de magnésium oxyéthylénés, le cocoyl iséthionate de sodium et les méthyl acyl taurate.

De préférence, le ou les tensioactifs anioniques sulfates ou sulfonates (ii) sont choisis parmi les alkyl(C₁₂-C₁₄)éthersulfates de sodium, en particulier le lauryléther sulfate de sodium, encore plus préférentiellement ceux comportant de 2 à 3 moles d'oxyde d'éthylène.

Les tensioactifs anioniques sulfates ou sulfonates (ii) sont généralement présents dans une teneur allant de 1 à 25% en poids, de préférence dans une teneur allant de 3 à 20% en poids, et plus préférentiellement dans une teneur allant 5 à 15% en poids, par rapport au poids total de la composition cosmétique.

La composition cosmétique comprend en outre un ou plusieurs tensioactifs anioniques carboxyliques (iii) différents des tensioactifs (ii) qui sont choisis parmi les acides alkyl éther carboxyliques et leurs sels.

Le ou les tensioactifs anioniques carboxyliques (iii) différents des tensioactifs anioniques (ii) ne comprennent de préférence pas de fonction sulfate ou sulfonate et sont choisis parmi les acides alkyl(C₆-C₂₄) éther carboxyliques polyoxyalkylénés et leurs sels, en particulier ceux comportant de 2 à 50 à groupements oxyde d'alkylène en particulier d'oxyde d'éthylène tels que les composés proposés par la sociétés KAO sous les dénominations AKYPO.

Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

Les tensioactifs anioniques carboxyliques (iii) sont en particulier choisis parmi les composés qui répondent à la formule (IV) suivante : 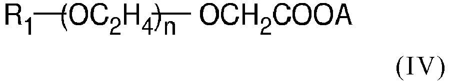 dans laquelle :
R₁ représente un radical ou un mélange de radicaux alkyle ou alcényle linéaire ou ramifié en C₈-C₂₂, un radical alkyl(C₈-C₉)phényle, un radical R₂CONH-CH₂-CH₂- avec R₂ désignant un radical alkyle ou alcényle linéaire ou ramifié en C₁₁-C₂₁, et
n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,
A désigne un atome d'hydrogène, un groupement ammonium, Na, K, Li, Mg ou un reste monoéthanolamine ou triéthanolamine et de préférence, un atome d'hydrogène ou de sodium, et plus particulièrement de sodium.

On peut également utiliser des mélanges de composés de formule (IV) en particulier des mélanges dans lesquels les groupements R₁ sont différents.

Préférentiellement, R₁ représente un radical alkyle ou alcényle linéaire ou ramifié en C₈-C₂₂, un radical alkyl(C₈-C₉)phényle.

Le ou les tensioactifs anioniques carboxyliques (iii) ou leurs sels utilisés dans la composition cosmétique selon l'invention sont de préférence choisis parmi ceux de formule (IV) dans laquelle R₁ désigne un radical ou un mélange de radicaux alkyl(C₁₂-C₁₄), cocoyle, oléyle; un radical nonylphényle ou octylphényle, A désigne un atome d'hydrogène ou de sodium, et n varie de 2 à 20 et de préférence 2 à 10.

Plus préférentiellement, le ou les tensioactifs anioniques carboxyliques (iii) sont choisis parmi les composés de formule (IV) dans laquelle R₁ désigne un radical alkyle en C₁₂-C₁₄ et, plus particulièrement en C₁₂, A désigne un atome d'hydrogène ou de sodium, en particulier le sodium, et n varie de 2 à 10.

Parmi les produits commerciaux, on peut utiliser de préférence les produits vendus par la société CHEM Y sous les dénominations :
AKYPO NP 70 (R=nonylphényle, n=7, A=H),
AKYPO NP 40 (R=nonylphényle, n=4, A=H)
AKYPO OP 40 (R=octylphényle, n=4, A=H)
AKYPO OP 80 (R=octylphényle, n=8, A=H)
AKYPO OP 190 (R=octylphényle, n=19, A=H)
AKYPO RLM 38 (R= alkyl(C₁₂-C₁₄), n=3,8 et A=H)
AKYPO RLM 38 NV (R= alkyl(C₁₂-C₁₄), n=4, A=Na)
AKYPO RLM 45 (R= alkyl(C₁₂-C₁₄), n=4,5 et A=H)
AKYPO RLM 45 NV (R= alkyl(C₁₂-C₁₄), n=4,5, et A=Na)
AKYPO RLM 100 (R= alkyl(C₁₂-C₁₄), n=10, et A=H)
AKYPO RLM 100 NV (R= alkyl(C₁₂-C₁₄), n=10, et A=Na)
AKYPO RLM 130 (R= alkyl(C₁₂-C₁₄), n=13, et A=H)
AKYPO RLM 160 NV (R= alkyl(C₁₂-C₁₄), n=16, et A=Na)
ou par la société SANDOZ sous les dénominations :
SANDOPAN DTC-Acid (R= alkyl(C₁₃), n=6, et A=H)
SANDOPAN DTC (R= alkyl(C₁₃), n=6, et A=Na)
SANDOPAN LS 24 (R= alkyl(C₁₂-C₁₄), n=12, et A=Na)
SANDOPAN JA 36 (R= alkyl(C₁₃), n=18, et A=H),
et plus particulièrement, les produits vendus sous les dénominations suivantes :
AKYPO RLM 45
AKYPO RLM 100
AKYPO RLM 38.

Encore plus préférentiellement, le tensioactif anionique carboxylique (iii) utilisé dans la composition selon l'invention correspond au produit vendu sous la dénomination AKYPO RLM 45.

Le ou les tensioactifs anioniques carboxyliques (iii) est ou sont présents dans une teneur allant de 0,05 à 10 %en poids, de préférence dans une teneur allant de 0,1 à 8% en poids, et plus préférentiellement dans une teneur allant de 0,2% à 5% en poids, par rapport au poids total de la composition.

Comme indiqué précédemment, la composition cosmétique selon l'invention comprend en outre un ou plusieurs tensioactifs amphotères.

Le ou les tensioactifs amphotères ou zwittérioniques utilisés dans la composition cosmétique selon la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (V) et (VI) :

Rₐ-CONHCH₂CH₂-N(R_{b})(R_{c})(CH₂COO⁻) (V)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (VI)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Le ou les tensioactifs amphotères peuvent être présents dans une teneur allant de 0,1 à 15% en poids, de préférence dans une teneur allant de 0,5 à 10% en poids, et encore plus préférentiellement dans une teneur allant de 1% à 8% en poids, par rapport au poids total de la composition cosmétique selon l'invention.

La composition cosmétique selon l'invention peut comprendre également un ou plusieurs tensioactifs additionnels choisis parmi les tensioactifs non ioniques.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁-₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels varie de préférence de 0,01 à 20% en poids, mieux encore de 0,1 à 10 % en poids par rapport au poids total de la composition.

De préférence, la quantité totale en tensioactifs dans la composition cosmétique selon l'invention varie de 3 à 50% en poids, plus préférentiellement de 5 à 30% en poids, mieux de 8 à 20% en poids, par rapport au poids total de la composition cosmétique.

La composition cosmétique peut également comprendre un ou plusieurs polymères cationiques.

Au sens de la présente invention, l'expression "polymère cationique" désigne tout polymère contenant des groupements cationiques et/ou des groupements ionisables en groupements cationiques.

Les polymères cationiques éventuellement présents dans la composition selon l'invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VII), (VIII), (IX) ou (X) suivantes: 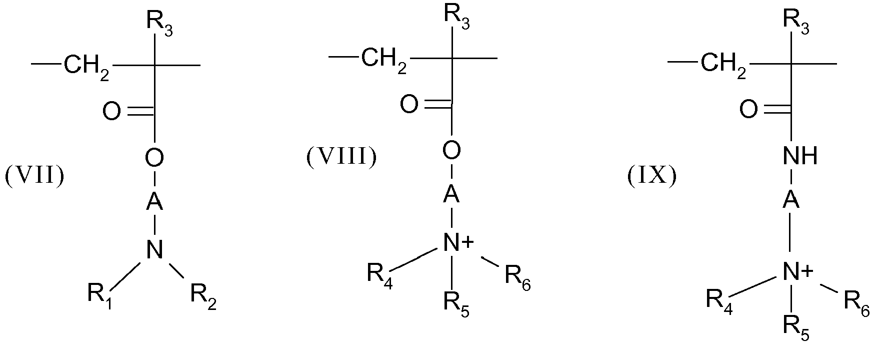 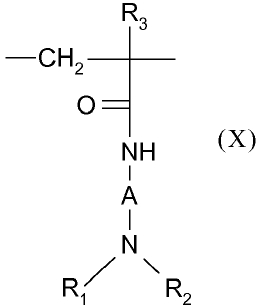 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   On peut citer en particulier l'homopolymère chlorure de méthacrylate d'éthyl triméthyl ammonium.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - les copolymères vinylpyrrolidone / méthacrylamide de diméthylaminopropyle quaternisés tel que le produit vendu sous la dénomination "GAFQUAT HS 100" par la société ISP,
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les dérivés d'éthers de cellulose comportant des groupements ammonium quaternaire décrits dans le brevet français 1 492 597, et en particulier les polymères commercialisés sous les dénominations "UCARE POLYMER JR" (JR 400 LT, JR 125, JR 30M) ou "LR" (LR 400, LR 30M) par la société AMERCHOL. Ces polymères sont également définis dans le dictionnaire CTFA comme des ammoniums quaternaires d'hydroxyéthylcellulose ayant réagi avec un époxyde substitué par un groupement triméthylammonium.
(3) Les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les gommes de guar cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (XI) ou (XII) : 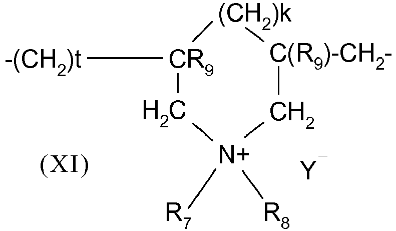 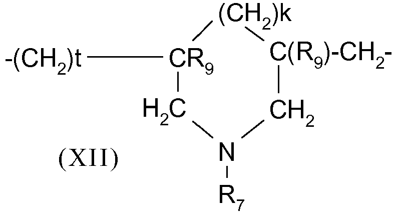 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faible masse moléculaire moyenne en poids) et les copolymères de chlorure de diallyldiméthylammonium et d'acrylamide commercialisés sous la dénomination "MERQUAT 550", MERQUAT 7SPR.
(10) Le polymère de diammonium quaternaire contenant des motifs récurrents répondant à la formule : 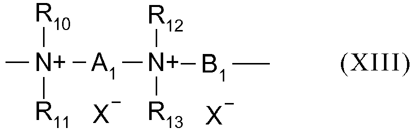 formule (XIII) dans laquelle :
   R₁₀, R₁₁, R₁₂ et R_{13,} identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 8 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XIV) suivante: 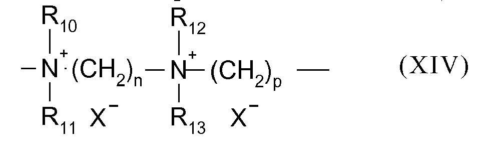 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 8 environ et, X⁻ est un anion dérivé d'un acide minéral ou organique. On peut citer en particulier le MEXOMERE PO commercialisé par la société CHIMEX.
(11) Les polyammoniums quaternaires constitués de motifs récurrents de formule (XV) : 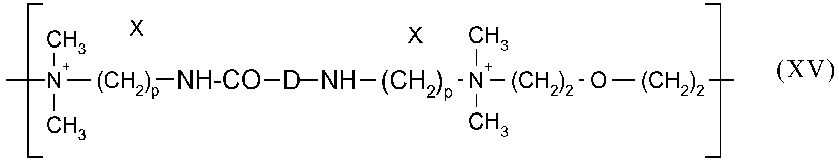 dans laquelle p désigne un nombre entier variant de 1 à 6 environ, D peut être nul ou peut représenter un groupement -(CH₂)ᵣ - CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F. Ces polymères peuvent également comprendre d'autres monomères comme les halogénures de diallyldialkylammonium. On peut citer, en particulier, le produit commercialisé sous la dénomination Luviquat Sensation par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA, ou les polyamines de coprah oxyéthyléné (15 OE).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (2), (3), (4), (10) et (12).

De préférence, le ou les polymères cationiques sont choisis parmi les celluloses cationiques, les gommes guar cationiques et les polymères quaternaires de vinylpyrrolidone et de vinyl imidazole éventuellement associé à d'autres monomères.

Plus préférentiellement, le ou les polymères cationiques sont choisis parmi les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyltriméthylammonium, de diméthyldiallyl ammonium, les gommes de guar cationiques le copolymère vinylpyrrolidone et de vinylimidazole et chlorure de diméthyldiallylammonium.

La teneur en polymère(s) cationique(s) dans la composition selon l'invention peut varier de 0,01 à 5% en poids par rapport au poids total de la composition, de préférence de 0,1% à 1% en poids, et plus préférentiellement de 0,15% à 0,5% en poids, par rapport au poids total de la composition.

La composition cosmétique selon l'invention peut également comprendre un ou plusieurs acides organiques.

Par acide organique, on entend tout composé organique non polymérique comportant une ou plusieurs fonctions acides choisies parmi les fonctions acide carboxylique, acide sulfonique, acide phosphorique.

Préférentiellement, l'acide organique n'est pas un tensioactif.

Encore plus préférentiellement, le poids moléculaire de l'acide organique est inférieur à 250, mieux inférieur à 200.

Les acides organiques peuvent être des acides aminés.

Le ou les acides organiques sont choisis de préférence parmi l'acide acétique, l'acide propanoïque, l'acide butanoïque, l'acide lactique, l'acide malique, l'acide glycolique, l'acide ascorbique, l'acide maléïque, l'acide phtalique, l'acide succinique, la taurine, l'acide tartrique, l'arginine, la glycine, l'acide glucuronique, l'acide gluconique et l'acide citrique.

Encore plus préférentiellement, les acides organiques selon l'invention sont les acides carboxyliques et mieux les acides carboxyliques alpha-hydroxylés ou AHA.

Encore plus préférentiellement encore, l'acide organique utilisé dans la composition selon l'invention est l'acide acétique, l'acide citrique et de préférence l'acide lactique.

Dans la composition, l'acide organique peut être sous forme libre ou salifiée.

Le ou les acides organiques pouvant être utilisés dans la composition selon la présente invention peuvent être présents en une teneur exprimée en acides libres allant de 0,01 à 10% en poids, de préférence en une teneur allant de 0,1 à 8% en poids, et encore plus préférentiellement en une teneur allant de 0,2 à 5% en poids, par rapport au poids total de la composition.

La composition cosmétique selon l'invention peut également comprendre une ou plusieurs silicones, de préférence aminées

Par « silicone aminée », on entend, au sens de la présente invention, toute silicone comportant au moins une fonction amine primaire, secondaire ou tertiaire ou un groupement ammonium quaternaire.

Les silicones aminées éventuellement utilisées dans la composition cosmétique selon la présente invention sont choisies parmi :
(a) les composés répondant à la formule (XVI) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (XVI)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :

      -N(R²)-CH₂-CH₂-N(R²)₂ ;

      -N(R²)₂ ; -N⁺(R²)₃ Q⁻ ;

      -N⁺(R²) (H)₂ Q⁻ ;

      -N⁺(R²)₂HQ⁻ ;

      -N(R²)-CH₂-CH₂-N⁺(R²)(H)₂ Q⁻,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
   En particulier, les silicones aminées correspondant à la définition de la formule (XVI) sont choisies parmi les composés correspondant à la formule suivante : 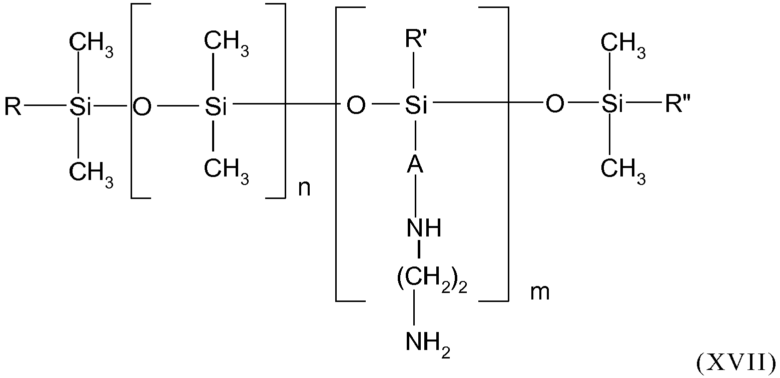 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
   Un produit correspondant à la définition de la formule (XVII) est en particulier le polymère dénommé dans le dictionnaire CTFA "triméthylsilylamodiméthicone", répondant à la formule (XIX) suivante: 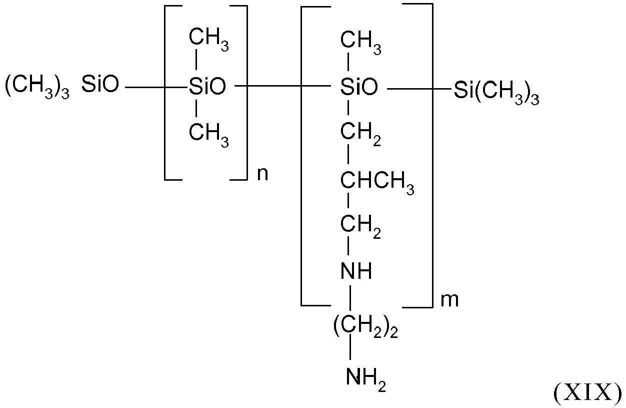 dans laquelle n et m ont les significations données ci-dessus conformément à la formule (XVII).
   De tels composés sont décrits par exemple dans EP 95238; un composé de formule (XIX) est par exemple vendu sous la dénomination Q2-8220 par la société OSI.
(b) les composés répondant à la formule (XX) suivante : 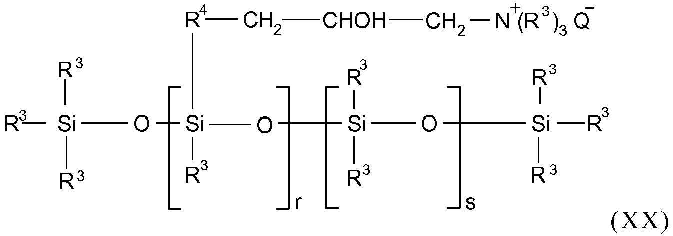 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

   De tels composés sont décrits plus particulièrement dans le brevet US 4185087.
   Un composé entrant dans cette classe est celui vendu par la Société Union Carbide sous la dénomination "Ucar Silicone ALE 56".
c) les silicones ammonium quaternaire de formule (XXI) : 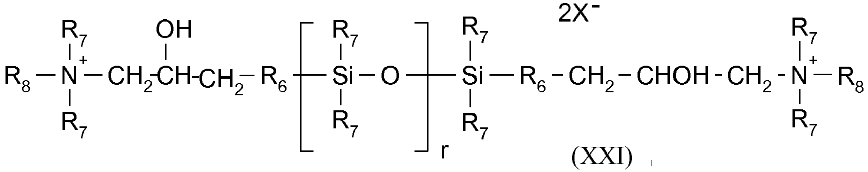 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

   Ces silicones sont par exemple décrites dans la demande EP-A-0530974.
d) les silicones aminées de formule (XXII) : 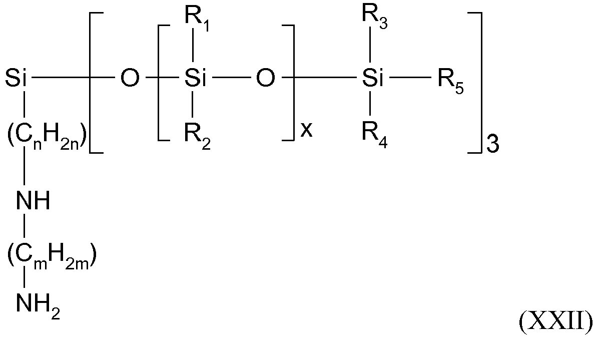 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

De préférence, les silicones aminées utilisées dans les compositions selon l'invention ne comprennent pas de groupements ammoniums quaternaires.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention varie généralement de 3 à 11 et de préférence de 5 à 10, mieux de 7 à 10.

La composition selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ ; des céramides ; des esters gras tels que le myristate d'isopropyle, le myristate de myristyle, le palmitate de cétyle et le stéarate de stéaryle ; des huiles minérales, végétales ou synthétiques telles que les α-oléfines ou l'huile d'avocat, l'huile de colza, l'huile de noyaux d'abricot, l'huile de camélina, l'huile de vaseline ; des vitamines ou provitamines ; des polymères cationiques ou amphotères ; des agents de stabilisation du pH, des conservateurs ; et des colorants.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

De préférence, les compositions cosmétiques de l'invention sont transparentes ou translucides, c'est-à-dire que ces compositions présentent une transmittance à 600 nanomètres supérieure à 85%, mieux supérieure à 90% et encore mieux supérieure à 94%.

Les compositions conformes à l'invention peuvent être utilisées comme shampooings pour le lavage et le conditionnement des cheveux et ils sont appliqués dans ce cas-là, de préférence, sur les cheveux humides dans des quantités efficaces pour les laver, et la mousse générée par massage ou friction avec les mains peut être ensuite éliminée après un éventuel temps de pause, par rinçage avec de l'eau, l'opération pouvant être répétée une ou plusieurs fois.

Un autre objet de l'invention est un procédé de traitement cosmétique des fibres kératiniques, telles que les cheveux, qui consiste à appliquer une quantité efficace d'une composition telle que décrite ci-dessus, sur lesdites fibres, et de préférence à rincer après un éventuel temps de pose.

En particulier, le procédé de traitement cosmétique des fibres kératiniques est un procédé de lavage et de conditionnement des fibres kératiniques, en particulier des cheveux.

L'exemple suivant sert à illustrer la présente invention.

### EXEMPLE

On prépare la composition (A) selon l'invention à partir des ingrédients indiqués dans le tableau ci-dessous dont les quantités sont exprimées en pour cent en poids en produit en l'état, par rapport au poids total de la composition.

| Composition | A (invention) |
|---|---|
| Acide lactique | 0,27 |
| Mélange de chloro-5-méthyl-2-iosthiazoline-4-one-3 / méthylisothiazoline-4-one-3 / chlorure et nitrate de magnésium en solution aqueuse ⁽¹⁾ | 0,1 |
| Oléate de propylène glycol polyéthoxylé (55 OE) et de propylène glycol en solution hydroglycolique ⁽²⁾ | 0,6 |
| Hydroxyéthyl cellulose quaternisée par le chlorure de 2,3 époxypropyl triméthyl ammonium à 95% M.A ⁽³⁾ | 0,6 |
| Polydiméthyl siloxane à groupements ⁽⁴⁾ aminoéthyl aminoisobutyle et triméthylsiloxy | 1 |
| 3-aminopropyltriéthoxysilane ⁽⁵⁾ | 0,75 |
| Cocoyl bétaïne à 30% M.A en solution aqueuse ⁽⁶⁾ | 17 |
| Alcool cétylique oxyéthyléné (20 OE) et oxypropyléné (5 OP) ⁽⁷⁾ | 0,5 |
| Acide lauryl éther carboxylique (4,5 OE) à 90% de matières actives dans l'eau ⁽⁸⁾ | 1 |
| Monoisopropanolamide d'acides de coprah à 94,5% de matières actives ⁽⁹⁾ | 0,85 |
| Lauryléther sulfate de sodium (2.2 OE) en solution aqueuse (70% M.A) ⁽¹⁰⁾ | 16 |
| Acide citrique ou hydroxyde de sodium | qs pH 9 |
| Parfum | 0,5 |
| Eau désionisée | qsp 100 g |

| | |
|---|---|
| ⁽¹⁾ vendu sous la dénomination commerciale KATHON CG par la société ROHM et HAAS ⁽²⁾ vendu sous la dénomination commerciale ANTIL 141 LIQUID par la société EVONIK GOLDSCHMIDT ⁽³⁾ vendu sous la dénomination commerciale POLYQUAT 400 KC par la société KCI ⁽⁴⁾ vendu sous la dénomination commerciale DC 28566 AMINOFLUID par la société DOW CORNING ⁽⁵⁾ vendu sous la dénomination XIAMETER OFS EO11 SILANE par la société DOW CORNING ⁽⁶⁾ vendu sous la dénomination MIRATAINER BB/FLA par la société RHODIA, ⁽⁷⁾ vendu sous la dénomination PROCETYL AWS-LQ par la société CRODA, ⁽⁸⁾ vendu sous la dénomination AKYPO RLM 45 CA par la société KAO, ⁽⁹⁾ vendu sous la dénomination EMPILAN CIS par la société HUNTSMAN ⁽¹⁰⁾ vendu sous la dénomination Texapon AOS 225UP par la société COGNIS | |

On obtient une composition qui est limpide et stable dans le temps.

Appliquée en tant que shampooing, la composition (A) apporte des effets coiffants satisfaisants, en particulier cette composition confère aux cheveux de la masse, du volume ainsi qu'un toucher doux satisfaisant.

## Revendications

1. Composition cosmétique pour le lavage et le conditionnement des fibres kératiniques, en particulier des fibres kératiniques humaines telles que les cheveux, comprenant dans un milieu cosmétiquement acceptable :
(i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques sulfates ou sulfonates,
(iii) un ou plusieurs tensioactifs anioniques carboxyliques différents des tensioactifs anioniques (ii) choisis parmi les acides alkyl éther carboxyliques ainsi que leurs sels, et
(iv) un ou plusieurs tensioactifs amphotères.

2. Composition cosmétique selon la revendication 1, **caractérisée en ce que** les fonctions chimiques basiques du composé organique du silicium sont choisies parmi les amines primaires, secondaires ou tertiaires.

3. Composition cosmétique selon la revendication 1 ou 2, **caractérisée en ce que** les groupes hydrolysables sont choisis parmi les groupes alcoxy, aryloxy et halogène.

4. Composition cosmétique selon l'une quelconque des revendications 1 à 3, **caractérisée en ce que** le ou les composés organiques du silicium sont choisis parmi les composés de formule (I) : 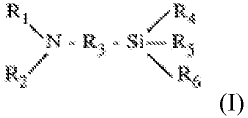 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR"', deux au moins des groupes R',
R" et R'" étant différents de l'hydrogène ; et 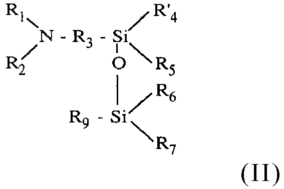
dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR"', OR₁₀ ou OR₈.

5. Composition cosmétique selon la revendication 4, **caractérisée en ce que** les groupes R₁, R₂, R', R'₁, R'₂, R'₃, R", R"', R"₁, R"₂, R₈, R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

6. Composition cosmétique selon l'une quelconque des revendications précédentes, **caractérisée en ce que** le ou les composés organiques du silicium sont choisis parmi les composés de formule (III) : 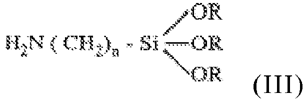 dans laquelle les radicaux R, identiques ou différents, sont choisis parmi les radicaux alkyle en C₁-C₆ et n est un nombre entier de 1 à 6, de préférence de 2 à 4.

7. Composition cosmétique selon l'une quelconque des revendications précédentes, **caractérisée en ce que** le ou les tensioactifs anioniques sulfates ou sulfonates (ii) sont choisis parmi les alkyl(C₁₂-C₁₄) sulfates de sodium, de triéthanolamine, de magnésium ou d'ammonium, les alkyl (C₁₂-C₁₄)éthersulfates de sodium, d'ammonium ou de magnésium, le cocoyl iséthionate de sodium et les méthyl acyl taurate.

8. Composition cosmétique selon l'une quelconque des revendications précédentes, **caractérisée en ce que** le ou les tensioactifs anioniques sulfates ou sulfonates (ii) sont choisis parmi les alkyl(C₁₂-C₁₄)éthersulfates de sodium.

9. Composition cosmétique selon l'une quelconque des revendications précédentes, **caractérisée en ce que** le ou les tensioactifs anioniques carboxyliques (iii) différents des tensioactifs anioniques (ii) sont choisis parmi les acides alkyl(C₆-C₂₄) éther carboxyliques polyoxyalkylénés et leurs sels, en particulier ceux comportant de 2 à 50 à groupements oxyde d'alkylène en particulier d'oxyde d'éthylène.

10. Composition cosmétique selon l'une quelconque des revendications précédentes, **caractérisée en ce que** le ou les tensioactifs anioniques carboxyliques (iii) sont choisis parmi les composés répondant à la formule (IV) suivante :
R₁(̵OC₂H₄)̵ₙ-OCH₂COOA (IV)
dans laquelle :
R₁ représente un radical ou un mélange de radicaux alkyle ou alcényle linéaire ou ramifié en C₈-C₂₂, un radical alkyl(C₈-C₉)phényle, un radical R₂CONH-CH₂-CH₂- avec R₂ désignant un radical alkyle ou alcényle linéaire ou ramifié en C₁₁-C₂₁, et, de préférence un radical alkyle ou alcényle linéaire ou ramifié en C₂-C₂₂, un radical alkyl(C₈-C₉)phényle,
n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,
A désigne un atome d'hydrogène, un groupement ammonium, Na, K, Li, Mg ou un reste monoéthanolamine ou triéthanolamine et de préférence, un atome d'hydrogène ou de sodium, et plus particulièrement de sodium.

11. Composition cosmétique selon l'une quelconque des revendications, **caractérisée en ce que** le ou les tensioactifs anioniques (iii) sont choisis parmi les composés répondant à la formule (IV) dans laquelle R₁ désigne un radical ou un mélange de radicaux alkyl(C₁₂-C₁₄), cocoyle, oléyle; un radical nonylphényle ou octylphényle, A désigne un atome d'hydrogène ou de sodium, et n varie de 2 à 20 et de préférence 2 à 10.

12. Composition cosmétique selon l'une quelconque des revendications précédentes, **caractérisée en ce que** le ou les tensioactifs amphotères sont choisis parmi les (alkyl en C₈₋₂₀)-bétaïnes, les (alkyl en C₈₋₂₀)-amido(alkyl en C₆₋₈)bétaïnes et leurs mélanges.

13. Composition cosmétique selon l'une quelconque des revendications précédentes, **caractérisée en ce qu'**elle comprend en outre un ou plusieurs polymères cationiques et/ou une ou plusieurs silicones, de préférence aminées.

14. Procédé de traitement cosmétique des fibres kératiniques, en particulier des fibres kératiniques humaines telles que les cheveux, **caractérisé en ce que** l'on applique la composition cosmétique telle que définie selon l'une quelconque des revendications précédentes, sur lesdites fibres, et **en ce que** l'on rince après un éventuel temps de pose.

15. Utilisation d'une composition selon l'une quelconque des revendications 1 à 13, comme shampooing pour le nettoyage et le conditionnement des fibres kératiniques.

## Patentansprüche

1. Kosmetische Zusammensetzung für das Waschen und die Konditionierung von Keratinfasern, insbesondere von menschlichen Keratinfasern wie etwa Haaren, welche in einem kosmetisch akzeptablen Medium umfasst:
(i) eine oder mehrere organische Siliciumverbindungen, die aus den ein Siliciumatom umfassenden Silanen und den zwei oder drei Siliciumatome umfassenden Siloxanen ausgewählt sind, wobei die organischen Siliciumverbindungen außerdem eine oder mehrere basische chemische funktionelle Gruppen und eine oder mehrere Hydroxygruppen oder hydrolysierbare Gruppen pro Molekül aufweisen;
(ii) ein oder mehrere anionische Tenside, die Sulfate oder Sulfonate sind,
(iii) ein oder mehrere carboxylische anionische Tenside, die von den anionischen Tensiden (ii) verschieden sind und aus den Alkylethercarboxylsäuren sowie deren Salzen ausgewählt sind, und
(iv) ein oder mehrere amphotere Tenside.

2. Kosmetische Zusammensetzung nach Anspruch 1, **dadurch gekennzeichnet, dass** die basischen chemischen funktionellen Gruppen der organischen Siliciumverbindung aus den primären, sekundären oder tertiären Aminen ausgewählt sind.

3. Kosmetische Zusammensetzung nach Anspruch 1 oder 2, **dadurch gekennzeichnet, dass** die hydrolysierbaren Gruppen aus den Alkoxy-, Aryloxy- und Halogengruppen ausgewählt sind.

4. Kosmetische Zusammensetzung nach einem der Ansprüche 1 bis 3, **dadurch gekennzeichnet, dass** die eine oder die mehreren organischen Siliciumverbindungen ausgewählt sind aus den Verbindungen der Formel (I): 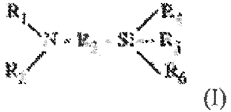 in der:
R₄ ein Halogen, eine Gruppe OR' oder R'₁ bedeutet;
Rs ein Halogen, eine Gruppe OR" oder R'₂ bedeutet;
R₆ ein Halogen, eine Gruppe OR''' oder R'₃ bedeutet;
R₁, R₂, R₃, R', R'', R''', R'₁, R'₂, R'₃ unabhängig voneinander eine gesättigte oder ungesättigte, lineare oder verzweigte Kohlenwasserstoffgruppe bedeuten, die eventuell zusätzliche chemische Gruppen trägt, wobei R₁, R₂, R', R'' und R''' außerdem Wasserstoff bezeichnen können und wenigstens zwei der Gruppen R₄, R₅ und R₆ OR', OR'' bzw. OR''' bezeichnen, wobei wenigstens zwei der Gruppen R', R'' und R''' von Wasserstoff verschieden sind; und 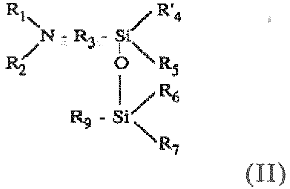
in der:
R₁, R₂, R₃, R₅ und R₆ wie zuvor definiert sind;
R'₄ ein Halogenatom oder eine Gruppe OR₁₁ bedeutet;
R₇ ein Halogenatom, eine Gruppe OR₁₀ oder R''₁ bedeutet;
R₉ ein Halogenatom, eine Gruppe OR₈, R''₂ oder R₃NR₁R₂ bedeutet;
R''₁, R''₂, R₈, R₁₀ und R₁₁ eine gesättigte oder ungesättigte, lineare oder verzweigte Kohlenwasserstoffgruppe bedeuten, die eventuell zusätzliche chemische Gruppen trägt, wobei die Gruppen R₁₁, R₁₀ und R₈ außerdem ein Wasserstoffatom bedeuten können; wobei wenigstens eine der Gruppen R₆, R₇ und R₉ ein Halogenatom, eine Gruppe OR''', OR₁₀ oder OR₈ bezeichnet.

5. Kosmetische Zusammensetzung nach Anspruch 4, **dadurch gekennzeichnet, dass** die Gruppen R₁, R₂, R', R'₁, R'₂, R'₃, R'', R''', R''₁, R''₂, R₈, R₁₀ und R₁₁ aus den C₁-C₁₂-Alkylradikalen, C₆ bis C₁₄-Arylradikalen, C₁ bis C₈-Alkyl-C₆ bis C₁₄-Arylradikalen und C₆ bis C₁₄-Aryl-C₁ bis C₈-Alkylradikalen ausgewählt sind.

6. Kosmetische Zusammensetzung nach einem der vorhergehenden Ansprüche, **dadurch gekennzeichnet, dass** die eine oder die mehreren organischen Siliciumverbindungen aus den Verbindungen der Formel (III) ausgewählt sind: 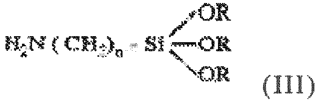 wobei die Radikale R, die identisch oder verschieden sind, aus den Alkylradikalen mit C₁-C₆ ausgewählt sind und n eine ganze Zahl von 1 bis 6 ist, vorzugsweise von 2 bis 4.

7. Kosmetische Zusammensetzung nach einem der vorhergehenden Ansprüche, **dadurch gekennzeichnet, dass** das eine oder die mehreren anionischen Tenside, die Sulfate oder Sulfonate sind (ii), aus den Alkyl (C₁₂-C₁₄) -sulfaten von Natrium, von Triethanolamin, von Magnesium oder von Ammonium, den Alkyl(C₁₂-C₁₄) -ethersulfaten von Natrium, von Ammonium oder von Magnesium, Natriumcocoylisethionat und den Methylacyltauraten ausgewählt sind.

8. Kosmetische Zusammensetzung nach einem der vorhergehenden Ansprüche, **dadurch gekennzeichnet, dass** das eine oder die mehreren anionischen Tenside, die Sulfate oder Sulfonate sind (ii), aus den Alkyl (C₁₂-C₁₄)-ethersulfaten von Natrium ausgewählt sind.

9. Kosmetische Zusammensetzung nach einem der vorhergehenden Ansprüche, **dadurch gekennzeichnet, dass** das eine oder die mehreren carboxylischen anionischen Tenside (iii), die von den anionischen Tensiden (ii) verschieden sind, aus den polyoxyalkylenierten Alkyl (C₆-C₂₄)-ethercarboxylsäuren und ihren Salzen ausgewählt sind, insbesondere denjenigen, die 2 bis 50 Alkylenoxidgruppen, insbesondere Ethylenoxidgruppen, aufweisen.

10. Kosmetische Zusammensetzung nach einem der vorhergehenden Ansprüche, **dadurch gekennzeichnet, dass** das eine oder die mehreren carboxylischen anionischen Tenside (iii) aus den Verbindungen ausgewählt sind, die der folgenden Formel (IV) entsprechen:
R₁(̵OC₂H₄)̵ₙ-OCH₂COOA (IV)
in der:
R₁ ein lineares oder verzweigtes Alkyl- oder Alkenylradikal mit C₈-C₂₂ oder ein Gemisch solcher Radikale, ein Alkyl(C₈-C₉)-phenylradikal, ein Radikal R₂CONH-CH₂-CH₂-, wobei R₂ ein lineares oder verzweigtes Alkyl- oder Alkenylradikal mit C₁₁-C₂₁ bezeichnet, und vorzugsweise ein lineares oder verzweigtes Alkyl- oder Alkenylradikal mit C₂-C₂₂, ein Alkyl(C₈-C₉)-phenylradikal, bedeutet,
n eine ganze Zahl oder Dezimalzahl (Mittelwert) ist, die von 2 bis 24 und vorzugsweise von 2 bis 10 variieren kann, wobei das Alkylradikal ungefähr zwischen 6 und 20 Kohlenstoffatome, vorzugsweise 8 bis 18 Kohlenstoffatome, aufweist und Aryl vorzugsweise Phenyl bezeichnet,
A ein Wasserstoffatom, eine Ammoniumgruppe, Na, K, Li, Mg oder einen Monoethanolamin- oder Triethanolaminrest und vorzugsweise ein Wasserstoff- oder Natriumatom und insbesondere ein Natriumatom bezeichnet.

11. Kosmetische Zusammensetzung nach einem der vorhergehenden Ansprüche, **dadurch gekennzeichnet, dass** das eine oder die mehreren anionischen Tenside (iii) aus den Verbindungen ausgewählt sind, die der Formel (IV) entsprechen, in der R₁ ein Alkyl (C₁₂-C₁₄)-radikal oder ein Gemisch solcher Radikale, Cocoyl, Oleyl; ein Nonylphenyl- oder Octylphenylradikal bezeichnet, A ein Wasserstoff- oder Natriumatom bezeichnet und n von 2 bis 20 und vorzugsweise von 2 bis 10 variiert.

12. Kosmetische Zusammensetzung nach einem der vorhergehenden Ansprüche, **dadurch gekennzeichnet, dass** das eine oder die mehreren amphoteren Tenside aus den (Alkyl mit C₈-C₂₀) -betainen, den (Alkyl mit C₈-C₂₀)-amido(alkyl mit C₆-C₈)-betainen und deren Gemischen ausgewählt sind.

13. Kosmetische Zusammensetzung nach einem der vorhergehenden Ansprüche, **dadurch gekennzeichnet, dass** sie außerdem ein oder mehrere kationische Polymere und/oder ein oder mehrere Silicone, vorzugsweise aminierte Silicone, umfasst.

14. Verfahren zur kosmetischen Behandlung von Keratinfasern, insbesondere von menschlichen Keratinfasern wie etwa Haaren, **dadurch gekennzeichnet, dass** die kosmetische Zusammensetzung, wie in einem der vorhergehenden Ansprüche definiert, auf diese Fasern aufgebracht wird, und dadurch, dass nach einer gegebenenfalls abgewarteten Einwirkzeit gespült wird.

15. Verwendung einer Zusammensetzung nach einem der Ansprüche 1 bis 13 als Shampoo zur Reinigung und Konditionierung der Keratinfasern.

## Claims

1. Cosmetic composition for washing and conditioning keratin fibres, in particular human keratin fibres such as the hair, comprising, in a cosmetically acceptable medium:
(i) one or more organosilicon compounds chosen from silanes comprising one silicon atom and siloxanes comprising two or three silicon atoms, the said organosilicon compounds also comprising one or more basic chemical functions and one or more hydroxyl or hydrolysable groups per molecule;
(ii) one or more sulfate or sulfonate anionic surfactants;
(iii) one or more carboxylic anionic surfactants other than the anionic surfactants (ii) chosen from alkyl ether carboxylic acids and salts thereof, and
(iv) one or more amphoteric surfactants.

2. Cosmetic composition according to Claim 1, **characterized in that** the basic chemical functions of the organosilicon compound are chosen from primary, secondary and tertiary amines.

3. Cosmetic composition according to Claim 1 or 2, **characterized in that** the hydrolysable groups are chosen from alkoxy, aryloxy and halogen groups.

4. Cosmetic composition according to any one of Claims 1 to 3, **characterized in that** the organosilicon compounds(s) are chosen from the compounds of formula (I): 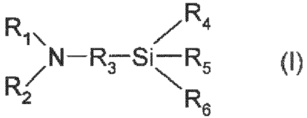 in which:
R₄ represents a halogen or a group OR' or R'₁;
R₅ represents a halogen or a group OR" or R'₂;
R₆ represents a halogen or a group OR"' or R'₃;
R₁, R₂, R₃, R', R", R"', R'₁, R'₂ and R'₃ represent, independently of each other, a saturated or unsaturated, linear or branched hydrocarbon-based group optionally bearing additional chemical groups, R₁, R₂, R', R" and R"' also possibly denoting hydrogen, and at least two of the groups R₄, R₅ and R₆ respectively denoting OR', OR" and OR"', at least two of the groups R', R" and R"' being other than hydrogen; and 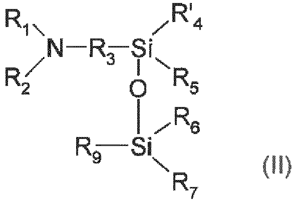
in which:
R₁, R₂, R₃, R₅ and R₆ are as defined previously;
R'₄ represents a halogen atom or a group OR₁₁;
R₇ represents a halogen atom or a group OR₁₀ or R''₁;
R₉ represents a halogen atom or a group OR₈, R"₂ or R₃NR₁R₂;
R"₁, R"₂, Rs, R₁₀ and R₁₁ represent a saturated or unsaturated, linear or branched hydrocarbon-based group, optionally bearing additional chemical groups, R₁₁, R₁₀ and R₈ also possibly representing a hydrogen atom; at least one of the groups R₆, R₇ and R₉ denoting a halogen atom or a group OR"', OR₁₀ or OR₈.

5. Cosmetic composition according to Claim 4, **characterized in that** the groups R₁, R₂, R', R'₁, R'₂, R'₃, R", R"', R"₁, R"₂, R₈, R₁₀ and R₁₁ are chosen from C₁-C₁₂ alkyl, C₆-C₁₄ aryl, (C₁-C₈)alkyl(C₆-C₁₄)aryl and (C₆-C₁₄)aryl(C₁-C₈)alkyl radicals.

6. Cosmetic composition according to any one of the preceding claims, **characterized in that** the organosilicon compound(s) are chosen from the compounds of formula (III): 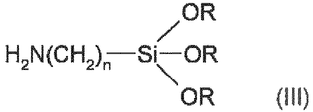 in which the radicals R, which may be identical or different, are chosen from C₁-C₆ alkyl radicals, and n is an integer from 1 to 6 and preferably from 2 to 4.

7. Cosmetic composition according to any one of the preceding claims, **characterized in that** the sulfate or sulfonate anionic surfactant(s) (ii) are chosen from sodium, triethanolamine, magnesium or ammonium (C₁₂-C₁₄)alkyl sulfates, sodium, ammonium or magnesium (C₁₂-C₁₄)alkyl ether sulfates, sodium cocoyl isethionate and methyl acyl taurates.

8. Cosmetic composition according to any one of the preceding claims, **characterized in that** the sulfate or sulfonate anionic surfactant(s) (ii) are chosen from sodium (C₁₂-C₁₄)alkyl ether sulfates.

9. Cosmetic composition according to any one of the preceding claims, **characterized in that** the carboxylic anionic surfactant(s) (iii) other than the anionic surfactants (ii) are chosen from polyoxyalkylenated (C₆-C₂₄)alkyl ether carboxylic acids and salts thereof, in particular those comprising from 2 to 50 alkylene oxide and in particular ethylene oxide groups.

10. Cosmetic composition according to any one of the preceding claims, **characterized in that** the carboxylic anionic surfactant(s) (iii) are chosen from compounds corresponding to formula (IV) below:
R₁-(OC₂H₄)ₙ-OCH₂COOA (IV)
in which:
R₁ represents a linear or branched C₈-C₂₂ alkyl or alkenyl radical or mixture of radicals, a (C₈-C₉)alkylphenyl radical, a radical R₂CONH-CH₂-CH₂- with R₂ denoting a linear or branched C₁₁-C₂₁ alkyl or alkenyl radical, and preferably a linear or branched C₂-C₂₂ alkyl or alkenyl radical or a (C₈-C₉)alkylphenyl radical,
n is an integer or decimal number (average value) that may range from 2 to 24 and preferably from 2 to 10, the alkyl radical containing between 6 and 20 carbon atoms approximately and preferably from 8 to 18 carbon atoms, and aryl preferably denoting phenyl,
A denotes a hydrogen atom, an ammonium group, Na, K, Li, Mg or a monoethanolamine or triethanolamine residue, preferably a hydrogen or sodium atom, more particularly a sodium atom.

11. Cosmetic composition according to any one of the preceding claims, **characterized in that** the anionic surfactant(s) (iii) are chosen from compounds corresponding to formula (IV) in which R₁ denotes a (C₁₂-C₁₄)alkyl, cocoyl or oleyl radical or mixture of radicals; a nonylphenyl or octylphenyl radical, A denotes a hydrogen or sodium atom, and n ranges from 2 to 20 and preferably 2 to 10.

12. Cosmetic composition according to any one of the preceding claims, **characterized in that** the amphoteric surfactant(s) are chosen from (C₈₋₂₀ alkyl)betaines and (C₈₋₂₀ alkyl)amido(C₆₋₈ alkyl)betaines, and mixtures thereof.

13. Cosmetic composition according to any one of the preceding claims, **characterized in that** it also comprises one or more cationic polymers and/or one or more silicones, preferably amino silicones.

14. Cosmetic process for treating keratin fibres, in particular human keratin fibres such as the hair, **characterized in that** the cosmetic composition as defined according to any one of the preceding claims is applied to the said fibres, and **in that** it is rinsed out after an optional leave-on time.

15. Use of a composition according to any one of Claims 1 to 13, as a shampoo for cleansing and conditioning keratin fibres.
